# FASCICULE DE BREVET EUROPEEN

(11) **EP 2 451 588 B1**
(45) Date de publication et mention de la délivrance du brevet: **04.03.2015**
(21) Numéro de dépôt: 10742225.5
(22) Date de dépôt: 07.07.2010
(51) Int. Cl.: A61F 13/00, A61L 15/18, B28B 1/32, A61K 9/00, B28B 19/00

(54) **SUBSTRAT IMPREGNE D'ARGILE PROJETE, SON PROCEDE DE FABRICATION ET SON UTILISATION**
MIT AUFGESPRÜHTEM TON IMPRÄGNIERTES SUBSTRAT, VERFAHREN ZU SEINER HERSTELLUNG UND SEINE VERWENDUNG
SUBSTRATE IMPREGNATED WITH SPRAYED CLAY, METHOD FOR MANUFACTURING SAME AND ITS USE

(30) Priorité: 07.07.2009 FR 0954696
(43) Date de publication de la demande: 16.05.2012
(73) Titulaire: Fontas, Valérie, 77410 Gressy En France (FR)
(72) Inventeur: MARTIN, Claude, F-10150 Sainte Maure (FR)
(74) Mandataire: Novagraaf Technologies
(86) Numéro de dépôt international: PCT/FR2010/051441
(87) Numéro de publication internationale: WO 2011/004126

(56) Documents cités:
- DE-A1-102007 049 429
- FR-A5- 2 096 036
- US-A1- 2007 276 308
- US-B1- 6 610 781

## Description

L'invention concerne la réalisation d'un support imprégné d'argile.

Il s'agit donc ici notamment de réaliser une pièce destinée à recouvrir une zone extérieure d'un organisme vivant, corps humain, animal ou végétal.

L'emploi de l'argile à des fins médicales ou de soins de beauté est connu depuis longtemps, et les propriétés thérapeutiques ou cosmétiques de certaines argiles ont déjà été étudiées.

A cet égard, en particulier dans EP-A-116 240, il a déjà été proposé une bande à charge active utilisable notamment à des fins de traitement chez l'homme et les animaux. Cette bande comprend un support à structure aérée pourtant une charge d'argile, en particulier d'argile verte, adhérant sur lui.

Pour réaliser de tels produits, il est connu de tremper un support plus ou moins aéré dans un bain d'argile. EP-A-116 240 y fait référence. Ce procédé a ses limites liées aux types de support utilisé et au mode d'apport de l'argile sur le support.

US 6610781, US2007/0276308 et FR2096036 font référence à des procédés d'imprégnation d'argile dans un support. US 6610781, US2007/0276308 envisagent une pulvérisation.

Il est ainsi connu :
- de réaliser un mélange pâteux contenant une charge comprenant (au moins) essentiellement de l'argile,
- de le pulvériser vers un support sec, souple à structure fibreuse,
- puis de sécher ledit support pourvu de ce mélange.

Aucun de ces documents ne fournit toutefois de détails réellement convaincant pour imprégner par pulvérisation.

D'ailleurs tant US6610781 que US2007/0276308 citent divers autres modes de chargement du support.

US2007/0276308 prévoit qu'avant d'être séché, le support imprégné soit roulé entre des rouleaux d'enfoncement ou gratte pour enfoncer davantage le mélange dans le support.

Et US6610781 nécessite que le mélange à mettre au contact du support comprenne un agent de polymérisation adapté à induire une polymérisation au sein du mélange ou en présence du support pourvu du mélange.

Ceci crée des conditions opératoires complexes et prouve que l'imprégnation par la seule pulvérisation n'est pas maîtrisée dans des conditions comparables à celle qui est ici proposée.

Un problème ici résolu est donc lié aux conditions pour réaliser par la seule pulvérisation l'imprégnation, par une charge à base (au moins essentiellement) d'argile, d'un support adapté, de façon que la charge dont le support séché est pourvu ne se sépare pas significativement de ce support lorsqu'il est ensuite immergé dans de l'eau distillée, à température ambiante.

Pour tendre vers ce but, il est prévu un procédé de réalisation selon la revendication 1, un produit selon la phylliteuse revendicaton 12 et l'utilisation d'un produit selon la revendication 21.

Ainsi, on va obtenir un produit fini de belle qualité (y compris de préférence sans irrégularité de bordure), avec une charge (éventuellement multi-composants) qui adhère bien, sans amas (bonne dispersion). Le produit fini peut aisément être mis sous des formes variées, sans rebus notable. On peut, si nécessaire, faire varier la quantité de charge, et passer rapidement d'une réalisation en bande enroulée à une série de masques, patchs (« rustines »), pansements, serviettes corporelles et autres.

A titre d'autres caractéristiques, à considérer seule ou en combinaisons partielles, on conseille aussi :
- que le mélange pulvérisé ait un taux de charge massique en argile inférieur à 42%,
- qu'on pulvérise contre une face seulement du support, en pouvant aller jusqu'à la saturer en charge, sans toutefois saturer ce support en argile sur toute son épaisseur,
- qu'on sèche ledit support imprégné sur un support d'appui qui le soutient du côté de sa face opposée à celle vers laquelle on a pulvérisé le mélange,
- qu'on pulvérise un mélange dépourvu de terres de diatomées,
- que le mélange pâteux réalisé contienne en masse entre 40% et 70% de liquide, en particulier de l'eau,
- que ledit support non-tissé (1) présente:
   * sec, non imprégné, une densité comprise entre 40 g/m² et 100 g/m² avant pulvérisation, et/ou une capacité d'absorption (test Edana) comprise entre 10 g/g et 18 g/g,
   * et entre 80 g/m² et 550 g/m² imprégné, donc après pulvérisation ;
- qu'on réalise le mélange pâteux à partir d'argile ayant une granulométrie entre 10 microns et 100 microns ;
- que ledit support comprenne exclusivement des fibres naturelles comprenant de la cellulose, le mélange à pulvériser comprenant exclusivement des composés naturels et étant ainsi dépourvu d'additif de maintien en suspension, une agitation du mélange à pulvériser étant alors établi pour placer l'argile en suspension,
- que l'argile soit une illite,
- qu'on pulvérise ledit mélange contre une face seulement du support, en chargeant davantage une face que la face opposée, sans saturer ce support sur toute son épaisseur, et en imprégnant cette charge dans et entre les fibres au moins localement, sur une partie au moins de ladite épaisseur.

Concernant le produit en lui-même, on prévoit dans le même sens que puisse être proposé un produit souple, enroulable, sec, comprenant donc un support airlaid poreux à fibres naturelles ou synthétiques et chargé (au moins) d'argile, cette charge étant dépourvue de réseau chimiquement intégré entre l'argile et un polymère et imprégnant le support de façon hétérogène sur son épaisseur, dans et entre les fibres au moins localement, avec au moins une non saturation d'une des faces, des espaces vides existant entre les fibres.

Si le produit est obtenu par la mise en oeuvre du procédé précité, les fibres de son support chargé seront au moins localement moins entremêlées que celles du support sec, non encore chargé, vers lequel on aura pulvérisé le mélange.

De façon générale, le produit en cause peut être aussi caractérisé avantageusement en ce que :
- les fibres du support chargé sont moins entremêlées du côté du support opposé à celui le plus chargé,
- ledit support non-tissé présente, imprégné, une densité comprise entre 80g/m² et 550 g/m²,
- le support est chargé par une argile phylliteuse, non gonflante et d'une granulométrie entre 20 microns et 30 microns, de préférence monophyllite et polyphyllite
- il est dépourvu de terres de diatomées.

D'autres caractéristiques et avantages apparaîtront encore de la description qui suit, faite sans visée limitative et en référence aux figures annexées où :
- la figure 1 montre une possibilité d'aspersion par pistolet manuel,
- la figure 2 schématise une réalisation de bande d'airlaid chargée par projection automatisée,
- les figures 3, 4 5, 6 et 7, 8 montrent par groupe de deux (horizontalement) des sections de support chargé (face pulvérisée à droite sur chaque figure), en coupe, sans préparation, sous microscope optique,
- les figures 9, 10, 11, 12 montrent par groupe de deux (horizontalement) deux sections de support chargé (face pulvérisée à gauche sur chaque figure), en coupe, avec section polie, sous microscope optique,
- les figures 13, 14, 15, 16 montrent une section de support chargé, en coupe, sous microscope à balayage, avec les fibres prises dans l'argile, et
- les figures 18, 19, 20, 21, 22 montrent des sections de support chargé (face pulvérisée à droite sur chaque figure), sous microscope à balayage.

Ce qui est présenté ci-après doit permettre que la charge 30 ne se sépare pas significativement du support 10 séché lorsqu'il est ensuite immergé dans de l'eau distillée, à température ambiante, comme s'il était utilisé par un acheteur.

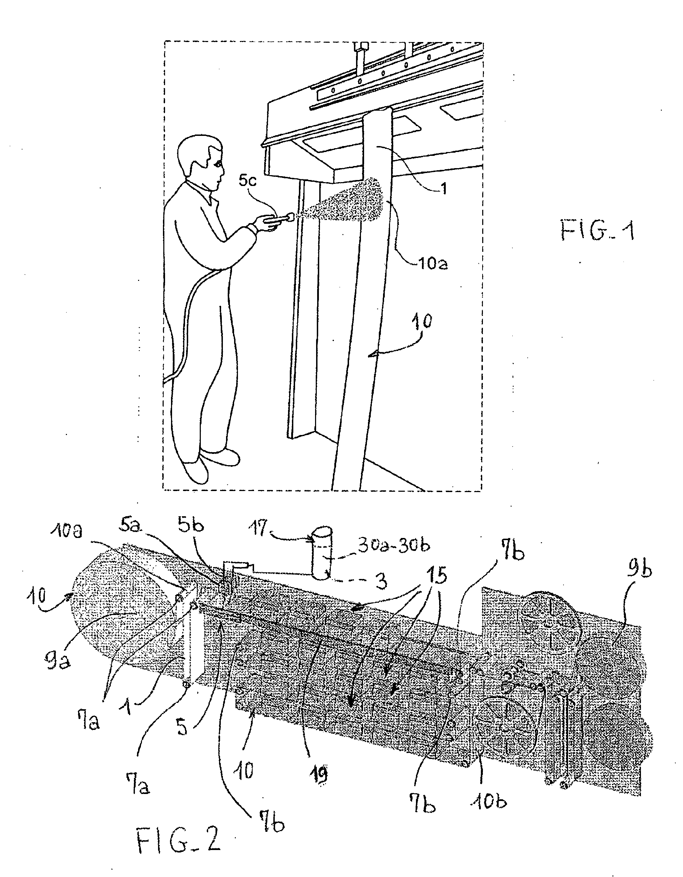

Dans ce but, et comme montré figures 1, 2, il est prévu de réaliser un support souple 10 imprégné (au moins) d'argile 30a (à coeur et en surface) en pulvérisant sur un support ou substrat vierge souple 1, à structure aérée, un produit pâteux 3 contenant de l'argile en mélange avec un liquide 30b.

Pour réaliser ce produit pâteux, l'argile est apportée sèche sous forme de particules.

Pour imprégner à coeur le support 1, le produit pâteux pulvérisé est une pâte contenant ici, en masse de mélange, entre 30 et 60% d'argile et entre 40 et 70% de liquide, en particulier eau, voire alcool, ou combinaison. L'argile 30a utilisée sera phylliteuse et non gonflante.

Un résultat particulièrement performant a été constaté avec une argile ayant une granulométrie entre 10 microns et 100 microns, et de préférence entre 20 microns et 30 microns. On recommande que l'argile à pulvériser soit de l'illite.

Des additifs de renforcement des propriétés de l'argile pourront être adjoints à ce mélange pâteux, tels extraits végétaux, aromes.... L'argile demeurera largement majoritaire ; plus de 90%.

Pour favoriser la projection, on conseille même que le produit pâteux 3 contienne un taux de charge massique en argile inférieur à 42%. En outre, dans le réservoir 17, on l'y aura maintenu en suspension favorablement par agitation (de préférence à des dispersants, tels le « darvan »™); agitateur tournant - bras- qui remue la pâte boueuse, à basse vitesse.

A noter que le mélange à pulvériser, et donc le support chargé obtenu, pourra sans inconvénient être dépourvu de terres de diatomées, contrairement aux recommandations de US2007/0276308.

En outre, le mélange sera dépourvu de tout agent de polymérisation adapté à induire une polymérisation au sein du mélange ou en présence du support pourvu du mélange pulvérisé. Du fait des caractéristiques ici retenues, il n'est en effet pas nécessaire de réaliser un composite argile-polymère en réseau comme dans US6610781 pour que la charge tienne au support dans les conditions attendues.

Pour favoriser la qualité des produits dans le cadre d'une utilisation dans le domaine alimentaire, d'hygiène ou de soin, ainsi que le recyclage des produits après utilisation, on recommande:
- que le support 1 vers lequel la pulvérisation est opérée comprenne exclusivement des fibres naturelles comprenant (de préférence exclusivement) de la cellulose,
- que le mélange à pulvériser comprenne exclusivement des composés naturels et soit ainsi dépourvu d'additif de maintien en suspension, l'agitateur précité permettant d'établir une agitation suffisante pour placer en suspension l'argile, et plus généralement la charge à projeter.

Pour la pulvérisation, il faut par ailleurs :
- que le mélange pâteux soit réalisé dans un réservoir 17 où on a établi une pression d'air comprise entre 1,5 et 7 X 10⁵ Pa,
- et/ou que le mélange soit pulvérisé à une distance du support comprise entré 5 cm et 30 cm.

Le cumul des deux caractéristiques assure une qualité de résultat.

Concernant les moyens d'apport, on voit figure 2 qu'il est prévu que la pulvérisation intervienne pardessus une bande de support 1 horizontale, avec des buses, telles 5a, 5b (figure 2), verticales projetant le mélange vers le bas et appartenant à des moyens pneumatiques 5 raccordés à l'intérieur du réservoir 17.

Une pression d'entrée d'air dans la/chaque buse de l'ordre de 1,8 à 2,5 10⁵ Pa est favorable. Des buses « devilbiss » (TM) fonctionnant sous 3 X 10⁵ Pa de pression d'air et de mélange boueux et des ajutages de 1,4 mm conviennent.

La possible version manuelle, comme figure 1, avec un opérateur manoeuvrant un pistolet de pulvérisation 5c dirigé vers le substrat 1 alors vertical n'est pas conseillée.

La pulvérisation s'opèrera vers un support maintenu sensiblement tendu.

Figure 1, des moyens de tension 7a, 7b et des bobines telles 9a, 9b, assurent alimentation en support, reprise et maintien tendu.

Favorablement, la pulvérisation s'opérera en chargeant davantage, en 30, une seule des faces (ici identiques) 10a du support, et sans saturer ce support sur toute son épaisseur.

Pour cela, la pulvérisation n'aura donc lieu que d'un côté du support.

Comme le montre les essais présentés plus loin, on conseille ainsi de ne pulvériser le mélange que vers une face seulement du support 10, jusqu'à la charger, a priori au moins jusqu'à 60 g/m2 d'argile, sans bien sur alors saturer la face opposée 10b, ni donc ce support sur son épaisseur, en imprégnant par contre la charge 30 dans et entre les fibres au moins localement, sur une partie au moins de cette épaisseur.

Pour obtenir un produit performant (bonne imprégnation et tenue de la charge, homogénéité en surface, qualité des découpes du produit chargé s'il y en a), on prévoit d'utiliser un substrat vierge 1 absorbant, tendu et sec, de type non-tissé à fibres naturelles ou synthétiques, obtenu par un procédé aérodynamique et voie sèche (airlaid).

Comme détaillé par exemple sous la référence internet « http://cerig.efpg.inpg.fr/tutoriel/non-tisse/ page03.htm », le procédé d'obtention d' « airlaid » agit par voie sèche et consiste à véhiculer et à disperser des fibres du type ci-dessus dans un flux d'air. Typiquement, on amène et fait passer les fibres à travers des cylindres rotatifs perforés ou des systèmes de distribution pour former un voile poreux, aéré, sur une toile transporteuse (caisse de distribution située au dessus d'une toile avec système de vide incorporé au dessous de la toile).

Pour atteindre dans les meilleures conditions, l'imprégnation recherchée, et en particulier les résultats fournis ci-après, on conseille que le support 1 présente:
- sec, non imprégné, une densité comprise entre 40 g/m² et 100 g/m² avant pulvérisation, et/ou une capacité d'absorption (test Edana) comprise entre 10 g/g et 18 g/g,
- et entre 80g/m² et 550 g/m² imprégné, après donc la pulvérisation.

La figure 2 montre qu'après la pulvérisation, on fera très avantageusement passer le support imprégné par la charge dans, ou entre, des moyens de séchage 15, étant précisé qu'avant cela, à la différence de ce qui enseigné dans US2007/0276308, le support imprégné n'aura pas été roulé (pressé) entre des rouleaux d'enfoncement ou gratter pour enfoncer davantage le mélange dans le support.

Pour la qualité de l'imprégnation recherchée, y compris au test du support chargé, séché, ensuite immergé dans de l'eau distillée, à température ambiante, on recommande de sécher le produit imprégné sur un support d'appui 19 (sensiblement horizontal) qui le soutient du côté de sa face 10b opposée à celle vers laquelle on a pulvérisé le mélange.

Les pertes d'une partie de la charge au séchage, et donc sur le produit fini, seront limitées, côté non pulvérisé 10b (ici donc côté inférieur).

Le séchage pourra comprendre des moyens de séchage, notamment un caisson, où le support chargé, ici la bande déroulante, sera séché à environ 80 à 200°C pendant 5 à 15 mn.

Ainsi, la bande chargée enroulée en 9b sera sèche.

Pour vérifier la qualité des résultats, des tests ont été menés, comme détaillé ci-dessous, avec examens de coupes de matériaux, sur tranche:

### Type d'analyses

- densités de support airlaid vierges entre 50g/m² et 85g/m², avec des épaisseurs entre 0,45mm et 1,4mm, sans préparation, puis après enrobage dans une résine transparente et polissage,
- microscope optique grossissement 25 à 500,
- matériaux (supports) analysés : airlaids vierges puis imprégnés,
- pistolets avec ajutage de 0 1,8 mm
- pressions dans réservoir à boue (17) : entre 1,5 et 7 X 10⁵ Pa,
- charges pulvérisées : argile phylliteuse, non gonflante (illite) représentant entre 30% et 41% en masse de mélange. Le liquide était de l'eau.
- Grammages mesurés :
   airlaid vierges : voir ci-dessus,
   airlaid imprégné : 490g/m² ;
   imprégnation par la charge projetée, dans les conditions précitées, sur une seule face du support 1.
- Remarque : ces mesures ont été effectuées sur des échantillons de 1 dm2 et ne tiennent pas compte des variations découlant d'une pulvérisation au pistolet manuel.

### Résultats :

### Observations sans préparation

- Support airlaid non imprégné : Fibres entremêlées ; Diamètre moyen d'une fibre : 20-30µm (typiquement 25 µm)
- Support imprégné : Epaisseur : plus faible (de l'ordre de -15 à plus de -20% (20.6%) par rapport au support airlaid vierge, sec) ; Argile dans l'épaisseur de l'airlaid ; Fibres mieux ordonnées, moins entremêlées que dans le support vierges, sec, au moins du côté opposé à celui où a eu lieu la pulvérisation.

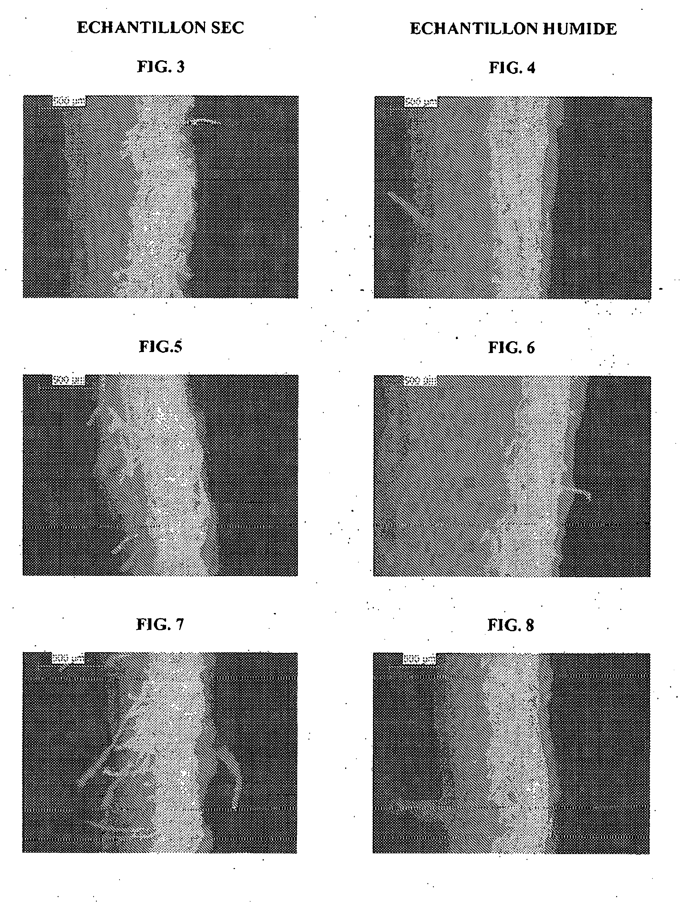

Il est constaté que l'échantillon sec (imprégné puis séché naturellement par appui sur un support du côté non pulvérisé) a perdu un peu de charge argileuse, mais rien de très significatif (voir figures 3, 5, 7 où les fibres sont bien imprégnées), ces « pertes » étant encore plus limitées ensuite lors de l'humidification du premier échantillon dans l'eau d'un bêcher (pendant 1 à 2 mn) ; voir figures 4, 6, 8.

### Observations avec préparations (inclusion dans un bloc résine poli).

- Support airlaid non imprégné : agencement désordonné des fibres, même diamètre moyen des fibres : 25 µm
- Support imprégné : Ordonnancement des fibres sur 300-450µm (côté pulvérisé), un peu moins face opposée ; Imprégnation totale du support, mais pas de saturation hors la face pulvérisée et sur environ 80-120µm.

### Conclusions :

- La charge d'argile est répartie dans l'épaisseur de l'airlaid (avec des gradients), bien que pulvérisée d'un côté seulement. La face opposée à la pulvérisation contient peu d'argile après séchage.
- Diminution d'épaisseur : La pression de pulvérisation induit une diminution d'épaisseur du matériau airlaid. Le retrait de l'argile lors du séchage peut être également une explication : on diminue la distance entre les feuillets de la microstructure silicatée.
- Ordonnancement et cohésion des fibres : Les fibres semblent se réarranger sous l'effet de la pression, de l'action du liquide de pulvérisation et du séchage final. L'ensemble devient cohésif.

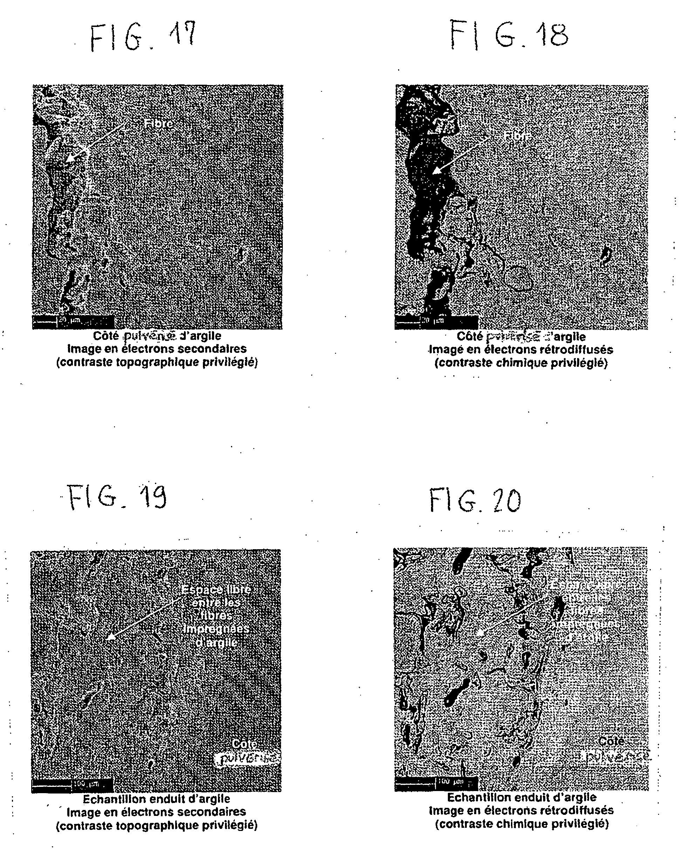

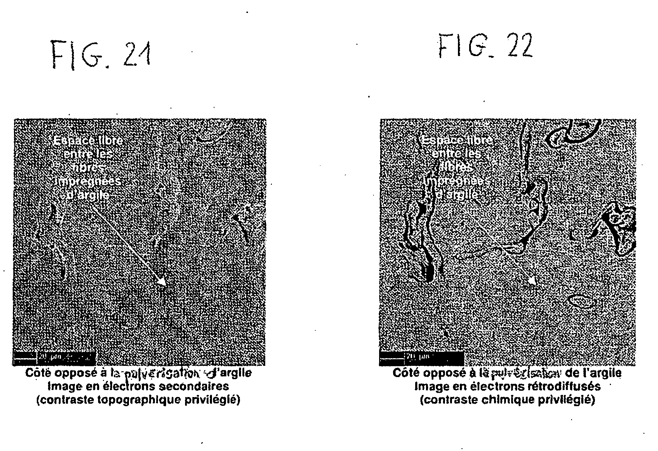

Les illustrations des figures 3-22 confirment ainsi ce qui précède, dans le cadre d'un support non-tissé airlaid, à fibres cellulosiques.

On voit que la charge à base argileuse imprègne le support de façon hétérogène sur son épaisseur, dans et entre les fibres au moins localement, avec une saturation du côté d'une des deux faces du support et sur une première partie de l'épaisseur, et une non saturation de la face opposée, où existent des espaces vides entre les fibres.

### Observations sans préparation (comme précédemment), audit microscope optique (figures 3-8) :

La diminution de l'épaisseur parait due à une meilleure cohésion des fibres entre elles grâce à l'imprégnation d'argile et à la pression induite par l'action de pulvérisation, qui permet aux fibres de se ré-agencer de façon plus ordonnée.

Après séchage les fibres à l'opposé du côté pulvérisé ne sont (presque) plus enrobées de particules argileuses.

On observe également qu'à l'opposé du côté saturé, les fibres semblent plus ordonnées.

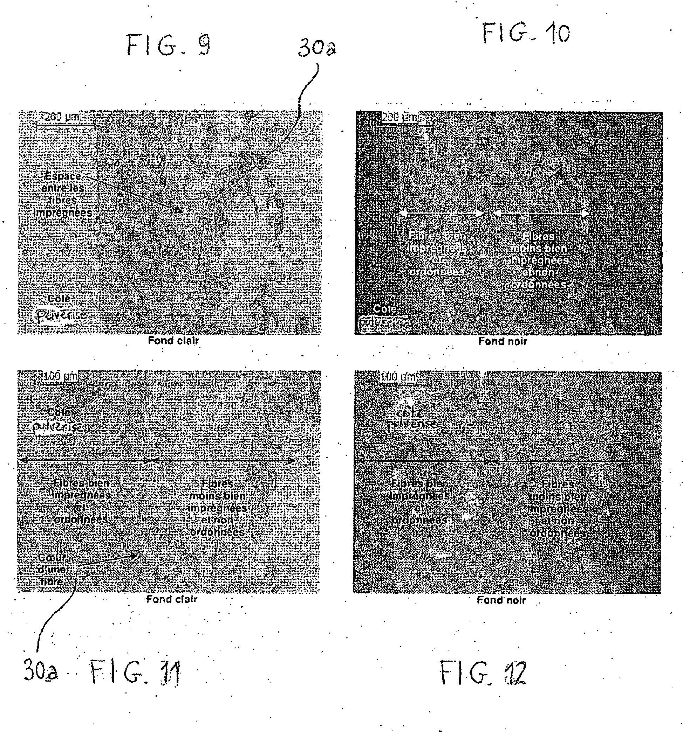

### Observations avec préparation/ inclusion dans un bloc résine poli (comme précédemment), avec ledit microscope optique ; voir figures 9 à 12:

Enduites d'argile, les fibres d'airlaid semblent ordonnées sur 370µm environ du côté imprégné : il s'agit des fibres les mieux enrobées d'argile. Dans cette partie, le contact entre l'argile et les fibres est intime : aucune porosité n'a été observée. Du côté non pulvérisé, on distingue des séparations entre fibres imprégnées d'argile : elles paraissent désordonnées.

### Conclusions partielles

Lorsque l'on pulvérise de l'argile sur le tissu de fibres cellulosiques, on constate que :
- les fibres sont recouvertes d'argile dans toute l'épaisseur du tissu ;
- les fibres à l'opposé du côté pulvérisé sont moins recouvertes de particules argileuses ;
- l'argile donne une cohésion à l'ensemble grâce à un contact intime entre les fibres et l'argile. La cohésion entre les fibres et l'argile ainsi que le fait de pulvériser ces particules argileuses permettent aux fibres cellulosiques de s'ordonner. De ce fait, l'épaisseur du tissu diminue.

Lorsque le tissu enduit d'argile est humidifié, les fibres les moins imprégnées d'argile perdent une partie de leurs particules argileuses. Cependant, les fibres gardent une certaine cohésion et l'épaisseur du tissu reste globalement identique.

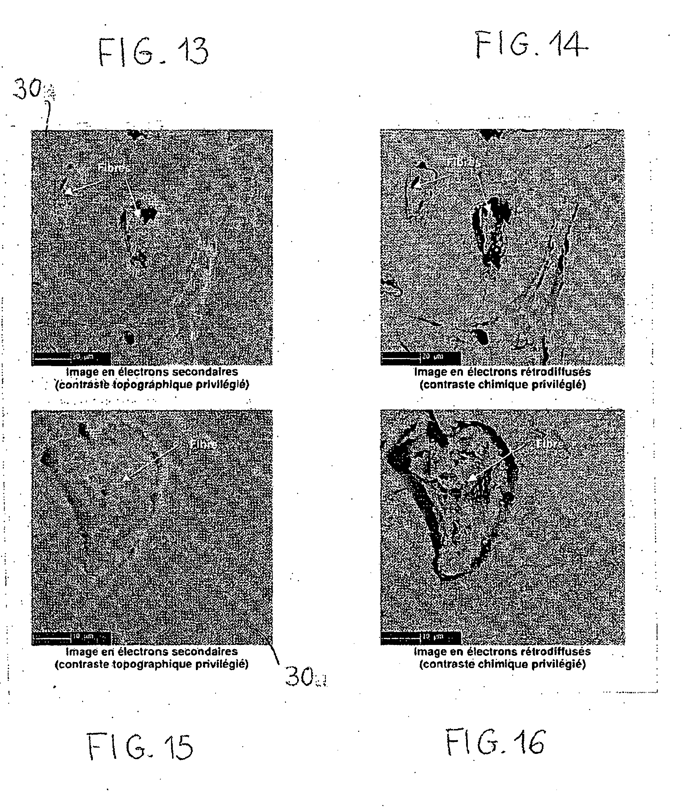

### Observations au microscope électronique (voir figures 13 à 22:

Mode opératoire:
1) Métallisation carbone d'une section polie précédentes
2) Observations en microscopie électronique à balayage.
L'observation en coupe d'un échantillon imprégné, de 500 µm d'épaisseur, permet de distinguer trois zones :
- la première zone, du côté où l'argile est pulvérisée sur le tissu, est constituée d'une couche très compacte de 100 µm environ ;
- la deuxième zone est constituée d'un amas moins compact de fibres recouvertes d'argile, sur 300 µm environ. Dans cet amas, on trouve des espaces entre les fibres ;
- la troisième zone, à l'opposé du côté d'imprégnation, est celle où l'on trouve des fibres éparses imprégnées d'argile (200 µm environ).

Chaque fibre observée en coupe est complètement imprégnée d'argile. Cependant, l'épaisseur de la couche d'argile varie : elle est plus importante du côté où l'argile a été pulvérisée. L'argile semble avoir pénétré au coeur des fibres, d'où un contact intime entre les deux produits.

### Conclusions :

Cette microscopie permet de préciser que l'argile s'est d'abord déposée dans le support airlaid, l'a saturé puis s'est empilée sur la face directe de pulvérisation. Ce dépôt n'est pas homogène dans l'épaisseur : dans un premier temps, on rencontre une couche compacte sans fibre de 100 µm environ, puis sur 300 µm, un mélange fibres/argile dans lequel les fibres sont totalement noyées dans l'argile (peu de manque matière). Enfin, une zone correspondant à la face opposée à la pulvérisation comporte plus de manque matière et n'est pas saturée en charge.

L'argile noie l'ensemble des fibres et colmate la surface exposée à la pulvérisation, entraînant ainsi la formation d'une couche de charge quasi-pure.

La pénétration de la charge au sein des fibres a provoqué un accrochage intime entre les fibres et la charge.

Ainsi, la charge argileuse reste emprisonnée dans l'airlaid séché (pénétration dans et entre les fibres) et s'est déposée en fine couche sur le support lors de l'humidification de l'échantillon (argile entre les fibres entraînées par le liquide de pulvérisation).

Dans des conditions similaires à celles ci-dessus, il a par ailleurs été vérifié que des résultats comparables peuvent être obtenus toujours en ne pulvérisant que sur une face du support airlaid, et en chargeant cette face pulvérisée avec 60 g/m², puis 180 g/m² d'argile, sans donc la saturer.

Une épaisseur de support chargés plus faible de - 5% à plus de -20% par rapport au support airlaid vierge, sec, est globalement noté.

Dans tous les cas, la charge ne se sépare en outre significativement pas du support chargé séché lorsqu'il est ensuite immergé dans de l'eau distillée, entre 3 s et 120 s, à température ambiante (entre 20°C et 30°C, typiquement 25°C). Ces tests menés, échantillons imprégnés puis séchés sous lampes, en appui côté non pulvérisé, avec ensuite des temps d'immersion complète dans de l'eau distillée pendant successivement 3 s, 5 s, 7 s, 15 s, 30 s n'ont pas révélés de séparation notable (significative) vis-à-vis du support airlaid de la charge, donc de l'argile, en particulier par dépôt dans l'eau.

Pour utiliser au mieux les qualités de l'argile en matière en particulier de conservation de produits agro-alimentaires, d'action par contact cutané, voire sous-cutané (plaie, performance hémostatique...), soin/beauté/hygiène (y compris hygiène intime), on conseille nettement, après les essais réalisés, que l'argile imprégnant le support non tissé airlaid 1 soit une illite (structure phylliteuse), avec dans sa composition 60 à 75% d'illite, 20 à 30% de smectite, 5 à 10% de kaolinite et divers éléments minéraux en traces (en particulier magnésium, potassium, zinc, cuivre et manganèse).

Ceci sera a priori complété par :
- un support 1 à fibres naturelles (cellulose), exclusivement,
- et une argile constituée intégralement d'un mélange naturel (non lessivé, ni traité au four, ni ionisé notamment) ayant une granulométrie entre 20 microns et 30 microns, et dont le diamètre moyen des pores sera de 70 à 75 Angströms.

Ainsi, cette illite (ici silicate d'aluminium hydraté d'origine naturelle, communément appelé argile verte) ayant une porosité de 0,11-0,13 cc/g, la surface spécifique permettra une adsorption optimale.

Pour les utilisations prévues, on notera encore que les argiles suivantes conviendront : argiles verte, rose, jaune, rouge.

Le domaine des produits à utilisation phytosanitaire (plantes, jardinage, etc.) et les applications de conservation de produits agro-alimentaires sont également visées et intéressantes.

Concernant les découpes du produit fini séché, elles sont possibles sans chauffer le moyen de coupe, sans effilochage. Le produit peut même se déchirer facilement, tout en restant suffisamment résistant pour les applications envisagées, surtout à l'état humide.

La couche visible d'argile déposée ne se désagrège pas pendant les opérations de bobinage, découpe et autres opérations de transformation (à sec), même si on plie le support chargé, qui demeure souple.

## Revendications

1. Procédé de réalisation d'un support imprégné d'argile (10), comprenant des étapes où :
- on réalise un mélange pâteux (3) contenant une charge comprenant essentiellement de l'argile (30a),
- on pulvérise vers un support sec, souple à structure fibreuse le mélange pâteux (3),
- puis on sèche ledit support pourvu de ce mélange,
**caractérisé en ce que**, de façon que la charge ne se sépare pas significativement du support séché lorsqu'il est ensuite immergé dans de l'eau distillée, à température ambiante :
- le mélange pâteux (3) qu'on réalise l'est avec de l'argile (30a) phylliteuse, non gonflante, représentant entre 30% et 60% en masse de mélange,
- le mélange pâteux est réalisé dans un réservoir où on a établi une pression d'air (17) comprise entre 1,5 et 7 X 10⁵ Pa et/ou le mélange est pulvérisé à une distance du support comprise entre 5 cm et 30 cm,
- ledit support vers lequel on pulvérise le mélange est tendu, de type non-tissé (1) à fibres naturelles ou synthétiques et obtenu par un procédé aérodynamique et voie sèche, le mélange que l'on pulvérise étant dépourvu d'agent de polymérisation adapté à induire une polymérisation au sein du mélange ou en présence du support pourvu du mélange pulvérisé,
- on pulvérise ledit mélange pour en imprégner le support,
- et, avant d'être séché, le support imprégné n'est pas roulé entre des rouleaux d'enfoncement ou gratter pour enfoncer davantage le mélange dans le support.

2. Procédé selon la revendication 1, **caractérisé en ce que** le mélange que l'on pulvérise a un taux de charge massique en argile inférieur à 42%.

3. Procédé selon l'une des revendications précédentes, **caractérisé en ce qu'**on pulvérise en chargeant davantage une face (10a) que la face opposée du support, sans saturer ce support sur toute son épaisseur.

4. Procédé selon la revendication 3, **caractérisé en ce que** l'on sèche ledit support imprégné sur un support d'appui qui le soutient du côté de sa face (10b) opposée à celle (10a) vers laquelle on a pulvérisé le mélange.

5. Procédé selon l'une des revendications précédentes, **caractérisé en ce qu'**on pulvérise un mélange dépourvu de terres de diatomées.

6. Procédé selon l'une des revendications précédentes, **caractérisé en ce que** le mélange pâteux (3) qu'on réalise contient en masse entre 40% et 70% de liquide (30b).

7. Procédé selon l'une des revendications précédentes, **caractérisé en ce que** ledit support non-tissé (1) présente:
- sec, non imprégné, une densité comprise entre 40g/m² et 100g/m² avant pulvérisation, et/ou une capacité d'absorption comprise entre 10g/g et 18g/g,
- et entre 80g/m² et 550g/m² imprégné, après donc pulvérisation.

8. Procédé selon l'une des revendications précédentes, **caractérisé en ce qu'**on réalise le mélange pâteux avec de l'argile ayant une granulométrie entre 20 microns et 30 microns.

9. Procédé selon l'une des revendications précédentes, **caractérisé en ce que** :
- ledit support non-tissé (1) vers lequel la pulvérisation est opérée comprend exclusivement des fibres naturelles comprenant de la cellulose,
- on pulvérise un mélange comprenant exclusivement des composés naturels et qui est ainsi dépourvu d'additif de maintien en suspension et on établit une agitation du mélange à pulvériser, pour placer l'argile en suspension.

10. Procédé selon l'une des revendications précédentes, **caractérisé en ce que** l'argile (30a) sélectionnée est une illite.

11. Procédé selon l'une des revendications précédentes, **caractérisé en ce qu'**on pulvérise ledit mélange contre une face (10a) seulement du support, en pouvant aller jusqu'à la saturation, sans de toutes façons saturer ainsi la face opposée ni une partie au moins de l'épaisseur du support, et en imprégnant la charge dans et entre les fibres au moins localement, sur une partie au moins de ladite épaisseur.

12. Produit souple, enroulable, sec, comprenant un support (1) adapté, poreux, non-tissé (1) à fibres naturelles ou synthétiques et chargé d'argile, ledit support étant imprégné de l'argile par une pulvérisation d'une charge à base d'argile, la charge imprégnant le support (1) de façon hétérogène sur son épaisseur, dans et entre les fibres au moins localement, avec au moins une non saturation en charge d'une des faces (10b) du support, des espaces vides existant entre les fibres, **caractérisé en ce que** le support (1) est de type obtenu par un procédé aérodynamique et voie sèche et est chargé au moins essentiellement d'argile, cette charge étant dépourvue de réseau chimiquement intégré entre l'argile et un polymère.

13. Produit selon la revendication 12, **caractérisé en ce qu'**une des faces (10b) du support est moins chargée au moins essentiellement d'argile que la face opposée (10a) et les fibres du support chargé (1) sont moins entremêlées du côté de cette face (10b) la moins chargée.

14. Produit selon l'une des revendications 12,13, **caractérisé en ce que** ledit support non-tissé (1) présente, imprégné, une densité comprise entre 80g/m² et 550 g/m².

15. Produit selon l'une des revendications 12, 13, **caractérisé en ce que** le support est au moins essentiellement chargé par une argile phylliteuse, non gonflante.

16. Produit selon l'une des revendications 12 à 15, **caractérisé en ce qu'**il est dépourvu de terres de diatomées.

17. Produit selon l'une des revendications 12 à 16, **caractérisé en ce que** l'argile est une illite.

18. Produit selon l'une des revendications 12 à 17, **caractérisé en ce que** l'argile est une illite comprenant 60 à 75% d'illite, 20 à 30% de smectite, 5 à 10% de kaolinite.

19. Produit selon l'une des revendications 12 à 16, **caractérisé en ce que** l'argile est une illite constituée intégralement d'un mélange naturel ayant une granulométrie entre 20 microns et 30 microns, et dont le diamètre moyen des pores est de 70 à 75 Angströms.

20. Produit selon l'une des revendications 12 à 19 pour son utilisation dans le traitement des plaies ou des hémorragies par contact cutané ou'sous-cutané.

21. Utilisation du produit selon l'une des revendications 12 à 19 pour son utilisation dans un soin de beauté ou un produit d'hygiène.

## Patentansprüche

1. Verfahren zur Herstellung eines Trägers, der mit Ton (10) imprägniert ist, umfassend die Schritte, in denen:
- ein pastöses Gemisch (3) hergestellt wird, das einen Füllstoff enthält, der im Wesentlichen Ton (30a) umfasst,
- das pastöse Gemisch (3) auf einen trockenen, flexiblen Träger mit faserartiger Struktur gesprüht wird,
- der mit diesem Gemisch versehene Träger dann getrocknet wird,
**dadurch gekennzeichnet, dass**, damit sich der Füllstoff nicht signifikant von dem getrockneten Träger löst, wenn er anschließend bei Umgebungstemperatur in destilliertes Wasser getaucht wird:
- das pastöse Gemisch (3) aus nicht quellfähigem, phyllitischem Ton (30a) hergestellt wird, der zwischen 30 % und 60 Gew.-% des Gemischs darstellt,
- das pastöse Gemisch in einem Tank hergestellt wird, in dem ein Luftdruck (17) im Bereich zwischen 1,5 und 7 X 10⁵ Pa aufgebaut wurde und/oder das Gemisch in einem Abstand von dem Träger im Bereich zwischen 5 cm und 30 cm gesprüht wird,
- wobei der Träger, auf den das Gemisch gesprüht wird, ein gespanntes Vlies (1) aus natürlichen oder synthetischen Fasern ist, das durch ein aerodynamisches und trockenes Verfahren erhalten wird, wobei das Gemisch, das gesprüht wird, frei von Polymerisierungsmittel ist, das geeignet ist, eine Polymerisation innerhalb des Gemischs oder in Gegenwart des mit dem gesprühten Gemisch versehenen Trägers zu induzieren,
- das Gemisch gesprüht wird, um damit den Träger zu imprägnieren,
- und der imprägnierte Träger, bevor er getrocknet wird, weder zwischen Druckwalzen gewalzt noch geschabt wird, um das Gemisch weiter in den Träger hineinzuarbeiten.

2. Verfahren nach Anspruch 1, **dadurch gekennzeichnet, dass** das Gemisch, das gesprüht wird, einen Füllstoffgewichtsanteil an Ton von weniger als 42 % aufweist.

3. Verfahren nach einem der vorhergehenden Ansprüche, **dadurch gekennzeichnet, dass** das Sprühen so erfolgt, dass eine Seite (10a) mehr befrachtet wird als die gegenüberliegende Seite des Trägers, ohne diesen Träger auf seiner gesamten Dicke zu sättigen.

4. Verfahren nach Anspruch 3, **dadurch gekennzeichnet, dass** der imprägnierte Träger auf einem Stützträger getrocknet wird, der ihn auf seiner Seite (10b) hält, die der Seite (10a), auf die das Gemisch gesprüht wurde, gegenüber liegt.

5. Verfahren nach einem der vorhergehenden Ansprüche, **dadurch gekennzeichnet, dass** ein Gemisch gesprüht wird, das frei von Diatomeenerden ist.

6. Verfahren nach einem der vorhergehenden Ansprüche, **dadurch gekennzeichnet, dass** das pastöse Gemisch (3), das hergestellt wird, zwischen 40 Gew.-% und 70 Gew.-% Flüssigkeit enthält (30b).

7. Verfahren nach einem der vorhergehenden Ansprüche, **dadurch gekennzeichnet, dass** der Vliesträger (1):
- trocken, nicht imprägniert, vor dem Besprühen eine Dichte im Bereich zwischen 40 g/m² und 100 g/m² und/oder eine Absorptionskapazität im Bereich zwischen 10 g/g und 18 g/g,
- und imprägniert, also nach dem Besprühen, zwischen 80 g/m² und 550 g/m² aufweist.

8. Verfahren nach einem der vorhergehenden Ansprüche, **dadurch gekennzeichnet, dass** das pastöse Gemisch mit Ton hergestellt wird, der eine Teilchengröße zwischen 20 Mikron und 30 Mikron aufweist.

9. Verfahren nach einem der vorhergehenden Ansprüche, **dadurch gekennzeichnet, dass**:
- der Vliesträger (1), der besprüht wird, ausschließlich natürliche Fasern umfasst, die Cellulose umfassen,
- ein Gemisch gesprüht wird, das ausschließlich natürliche Verbindungen umfasst und das daher frei von Suspensionsadditiv ist und das zu sprühende Gemisch gerührt wird, um den Ton in Suspension zu bringen.

10. Verfahren nach einem der vorhergehenden Ansprüche, **dadurch gekennzeichnet, dass** der ausgewählte Ton (30a) ein Illit ist.

11. Verfahren nach einem der vorhergehenden Ansprüche, **dadurch gekennzeichnet, dass** das Gemisch nur auf eine Seite (10a) des Träger gesprüht wird, wobei dies bis zur Sättigung erfolgen kann, wobei jedoch weder die gegenüberliegende Seite noch mindestens ein Teil der Dicke des Trägers auf diese Weise gesättigt werden und wobei der Füllstoff in und zwischen den Fasern mindestens lokal auf mindestens einem Teil der Dicke imprägniert wird.

12. Flexibles, rollbares, trockenes Produkt, umfassend einen geeigneten, porösen Träger (1) aus Vlies aus natürlichen oder synthetischen Fasern und mit Ton befrachtet, wobei der Träger mit Ton durch ein Sprühen eines Füllstoffs auf Tonbasis imprägniert wird, wobei der Füllstoff den Träger (1) auf seiner Dicke heterogen in und zwischen den Fasern mindestens lokal imprägniert, mit mindestens einer Nicht-Sättigung mit Füllstoff einer der Seiten (10b) des Trägers, wobei leere Räume zwischen den Fasern vorhanden sind, **dadurch gekennzeichnet, dass** der Träger (1) ein Träger ist, der durch ein aerodynamisches und trockenes Verfahren erhalten wird und mindestens im Wesentlichen mit Ton befrachtet ist, wobei dieser Füllstoff frei von einem chemisch integrierten Netzwerk zwischen dem Ton und einem Polymer ist.

13. Produkt nach Anspruch 12, **dadurch gekennzeichnet, dass** eine der Seiten (10b) des Trägers im Wesentlichen mindestens weniger mit Ton befrachtet ist als die gegenüberliegende Seite (10a) und dass die Fasern des befrachteten Trägers (1) auf dieser am wenigsten befrachteten Seite (10b) weniger verschränkt sind.

14. Produkt nach einem der Ansprüche 12, 13, **dadurch gekennzeichnet, dass** der imprägnierte Vliesträger (1) eine Dichte im Bereich zwischen 80 g/m² und 550 g/m² aufweist.

15. Produkt nach einem der Ansprüche 12, 13, **dadurch gekennzeichnet, dass** der Träger mindestens im Wesentlichen mit einem nicht quellfähigen, phyllitischen Ton befrachtet ist.

16. Produkt nach einem der Ansprüche 12 bis 15, **dadurch gekennzeichnet, dass** es frei von Diatomeenerden ist.

17. Produkt nach einem der Ansprüche 12 bis 16, **dadurch gekennzeichnet, dass** der Ton ein Illit ist.

18. Produkt nach einem der Ansprüche 12 bis 17, **dadurch gekennzeichnet, dass** der Ton ein Illit ist, umfassend 60 bis 75 % Illit, 20 bis 30 % Smektit, 5 bis 10 % Kaolinit.

19. Produkt nach einem der Ansprüche 12 bis 16, **dadurch gekennzeichnet, dass** der Ton ein Illit ist, der vollständig aus einem natürlichen Gemisch besteht, das eine Teilchengröße zwischen 20 Mikron und 30 Mikron aufweist und dessen mittlerer Porendurchmesser bei 70 bis 75 Ångström liegt.

20. Produkt nach einem der Ansprüche 12 bis 19 zur Verwendung bei der Behandlung von Wunden oder Hämorrhagien durch kutanen oder subkutanen Kontakt.

21. Verwendung des Produkts nach einem der Ansprüche 12 bis 19 zur Verwendung in der Schönheitspflege oder einem Hygieneprodukt.

## Claims

1. Process for the preparation of a support impregnated with clay (10), comprising stages where:
- a pasty mixture (3) comprising a filler essentially comprising clay (30a) is prepared,
- the pasty mixture (3) is sprayed onto a dry flexible support having a fibrous structure,
- then the said support provided with this mixture is dried,
**characterized in that**, so that the filler does not significantly separate off from the dry support when the support is subsequently immersed in distilled water, at ambient temperature:
- the pasty mixture (3) which is prepared is prepared with non-swelling phyllitic clay (30a) representing between 30% and 60% by weight of mixture,
- the pasty mixture is prepared in a tank where a pressure of air (17) of between 1.5 and 7 x 10⁵ Pa has been established and/or the mixture is sprayed at a distance from the support of between 5 cm and 30 cm,
- the said support onto which the mixture is sprayed is taut, of nonwoven type (1) comprising natural or synthetic fibres and obtained by an aerodynamic and dry-route process, the mixture which is sprayed being devoid of polymerization agent capable of bringing about polymerization within the mixture or in the presence of the support provided with the sprayed mixture,
- the said mixture is sprayed in order to impregnate the support with it,
- and, before being dried, the impregnated support is not rolled between ramming rollers or scraped in order to further ram the mixture into the support.

2. Process according to Claim 1, **characterized in that** the mixture which is sprayed has a content of filler by weight as clay of less than 42%.

3. Process according to either of the preceding claims, **characterized in that** spraying is carried out while charging one face (10a) more than the opposite face of the support, without saturating the support over its entire thickness.

4. Process according to Claim 3, **characterized in that** the said impregnated support is dried on a bearing support which supports it on the side of its face (10b) opposite that (10a) onto which the mixture has been sprayed.

5. Process according to one of the preceding claims, **characterized in that** a mixture devoid of diatomaceous earths is sprayed.

6. Process according to one of the preceding claims, **characterized in that** the pasty mixture (3) which is prepared comprises, by weight, between 40% and 70% of liquid (30b).

7. Process according to one of the preceding claims, **characterized in that** the said nonwoven support (1) exhibits:
- dry, nonimpregnated, a density of between 40 g/m² and 100 g/m² before spraying and/or an absorption capacity of between 10 g/g and 18 g/g,
- and between 80 g/m² and 550 g/m² impregnated, thus after spraying.

8. Process according to one of the preceding claims, **characterized in that** the pasty mixture is produced with clay having a particle size between 20 microns and 30 microns.

9. Process according to one of the preceding claims, **characterized in that**:
- the said nonwoven support (1) onto which the spraying is carried out comprises exclusively natural fibres comprising cellulose,
- the mixture comprising exclusively natural compounds and which is thus devoid of additive for holding in suspension is sprayed and stirring of the mixture to be sprayed is established in order to place the clay in suspension.

10. Process according to one of the preceding claims, **characterized in that** the clay (30a) selected is an illite.

11. Process according to one of the preceding claims, **characterized in that** the said mixture is sprayed against only one face (10a) of the support, being able to range up to saturation, without in any case thus saturating the opposite face or a portion at least of the thickness of the support, and impregnating the filler in and between the fibres, at least locally, over a portion at least of the said thickness.

12. Dry windable flexible product comprising a suitable porous non-woven support (1) having natural or synthetic fibres and possessing clay as filler, the said support being impregnated with the clay by spraying a clay-based filler, the filler impregnating the support (1) heterogeneously over its thickness, in and between the fibres at least locally, with at least one nonsaturation with filler of one of the faces (10b) of the support, empty spaces existing between the fibres, **characterized in that** the support (1) is of the type obtained by an aerodynamic and dry-route process and comprises at least essentially clay as filler, this filler being devoid of a network chemically incorporated between the clay and a polymer.

13. Product according to Claim 12, **characterized in that** one of the faces (10b) of the support has less filler at least essentially in the form of clay than the opposite face (10a) and the fibres of the filler-comprising support (1) are less interlaced on the side of this face (10b) comprising less filler.

14. Product according to either of Claims 12 and 13, **characterized in that** the said non-woven support (1) exhibits, impregnated, a density of between 80 g/m² and 550 g/m².

15. Product according to either of Claims 12 and 13, **characterized in that** the support has at least essentially as filler a non-swelling phyllitic clay.

16. Product according to one of Claims 12 to 15, **characterized in that** it is devoid of diatomaceous earths.

17. Product according to one of Claims 12 to 16, **characterized in that** the clay is an illite.

18. Product according to one of Claims 12 to 17, **characterized in that** the clay is an illite comprising from 60% to 75% of illite, from 20% to 30% of smectite and from 5% to 10% of kaolinite.

19. Product according to one of Claims 12 to 16, **characterized in that** the clay is an illite entirely composed of a natural mixture having a particle size of between 20 microns and 30 microns, the mean diameter of the pores of which is from 70 to 75 angstroms.

20. Product according to one of Claims 12 to 19 for the use thereof in the treatment of wounds or haemorrhages by cutaneous or subcutaneous contact.

21. Use of the product according to one of Claims 12 to 19 for the use thereof in a beauty care product or a hygiene product.
